Europäisches Patentamt

European Patent Office

Office européen des brevets

(11) Numéro de publication : **0 316 322 B1**

# (12) FASCICULE DE BREVET EUROPEEN

(45) Date de publication du fascicule du brevet :
**09.09.92 Bulletin 92/37**

(51) Int. Cl.$^5$ : **C07K 1/00**, C07K 5/10, C07K 7/00, A61K 37/02

(21) Numéro de dépôt : **87904695.1**

(22) Date de dépôt : **16.07.87**

(86) Numéro de dépôt international :
**PCT/FR87/00281**

(87) Numéro de publication internationale :
**WO 88/00594 28.01.88 Gazette 88/03**

(54) **TETRAPEPTIDE INHIBITEUR DE L'ENTREE EN CYCLE DES CELLULES SOUCHES HEMATOPO ETIQUES, PROCEDES POUR SON OBTENTION ET SES APPLICATIONS.**

(30) Priorité : **18.07.86 FR 8610486**

(43) Date de publication de la demande :
**24.05.89 Bulletin 89/21**

(45) Mention de la délivrance du brevet :
**09.09.92 Bulletin 92/37**

(84) Etats contractants désignés :
**AT BE CH DE FR GB IT LI LU NL SE**

(56) Documents cités :
EP-A- 0 121 912
WO-A-86/02381
Chemical Abstracts, vol. 97, 1982, (Columbus, Ohio, US), S.S. Wang et al.: "Synthesis of thymosin b 4", see pages 694-695,abstract 5623b, & Pept.:
Synth.Struc.,Funct.,Proc.Am.Pept.Symp., 7th 1981, 189-91
Chemical Abstracts, vol. 105, 1986, (Columbus 1-3,6, Ohio, US) see page 864, abstract 227323t, & JP, A, 6185399 (OTSUKAPHARMA-CEUTICAL CO., LTD) 30 April 1986
Chemical Abstracts, vol. 102, 1985, (Columbus, 1-3,6 Ohio, US), see page 397, abstract 191146j, & JP, A, 6008226 (OtsukaPharmaceu-tical Co., Ltd) 28June 1983

(73) Titulaire : **INSTITUT NATIONAL DE LA SANTE ET DE LA RECHERCHE MEDICALE (INSERM)**
**101, rue de Tolbiac**
**F-75654 Paris Cédex 13 (FR)**
Titulaire : **Institut Gustave Roussy**
**39-53, rue Camille Desmoulins**
**F-94800 Villejuif (FR)**

(72) Inventeur : **FRINDEL, Emilia**
**8, rue Meissonier**
**F-75017 Paris (FR)**
Inventeur : **LENFANT, Maryse**
**18, rue des Châtaigners**
**F-91190 Gif sur Yvette (FR)**
Inventeur : **GUIGON, Martine**
**37, rue de Chézy**
**F-92000 Neuilly (FR)**
Inventeur : **BAKALA, Johanna**
**9, avenue Saint-Mandé**
**F-75012 Paris (FR)**

(74) Mandataire : **Lachassagne, Jacques**
**Boite Postale 07**
**F-78430 Louveciennes (FR)**

EP 0 316 322 B1

## Description

L'invention a pour objet un nouveau tétrapeptide agissant en tant qu'inhibiteur de l'entrée en cycle des cellules souches hématopoïétiques ainsi que ses dérivés usuels dans le domaine des peptides à activité biologique.

L'invention a également pour objet un procédé pour l'extraction de ce tétrapeptide à partir de substances biologiques, notamment de moelle de veau foetal et un procédé pour sa synthèse, ainsi que celle de ses dérivés de substitution, par voie chimique.

Elle a en outre pour objet les applications de ce peptide et de ses dérivés de substitution en biologie et en médecine, notamment dans la protection de la moelle osseuse au cours de traitements anticancéreux par la chimiothérapie.

L'utilisation des médicaments dans le traitement des cancers est limitée par leurs effets toxiques sur les tissus sains, et en particulier, sur le tissu hématopoïétique. L'usage répété de ces drogues entraîne dans un grand nombre de cas soit une aplasie médulaire létale, soit une leucémie secondaire, soit des séquelles hématologiques moins graves.

Comme la plupart des médicaments anticancéreux n'agissent que sur les cellules en prolifération, les demandeurs ont pensé qu'il était possible de prévenir des dommages hématologiques en protégeant les cellules souches pluripotentes par des inhibiteurs de la prolifération.

Les travaux effectués par les demandeurs et publiés précédemment (voir notamment E. Frindel et M. Guigon, Exp. Hemat., 5 (1977), 74-76, M. Guigon et E. Frindel, Bull. Cancer 68(2), (1981), 150-153, J. Wdzieczak-Bakala, M. Lenfant et M. Guigon, IRCS Med. Sci., 12 (1984), 868-869, J. Wdzieczak-Bakala, M. Guigon, M. Lenfant et E. Frindel, Biomed. Pharm., 37 (1983), 467-471, et M. Guigon, J. Wdzieczak-Bakala, J.Y. Mary et M. Lenfant, Cell Tissue Kinet. 17 (1984), 49-55) ont montré qu'il était possible de réduire d'une façon significative, par administration d'un inhibiteur spécifique des cellules souches, la létalité observée chez les animaux au cours de traitements par la cytosine arabinoside (Ara-C), un médicament couramment utilisé en chimiothérapie du cancer, dont l'utilisation est limitée par son effet nocif sur la moelle osseuse. Cet inhibiteur spécifique qui est extrait de certaines substances biologiques, notamment de moelle osseuse ou de foie de foetus de veau, protège les cellules souches qui sont à l'origine de toutes les lignées sanguines.

En effet, les études biologiques effectuées montrent que l'augmentation de survie observée peut être attribuée à une protection des cellules souches hémopoïétiques, CFU-S (Colony Forming Units in the Spleen : unités formant des colonies dans la rate), c'est-à-dire des cellules souches pluripotentes capables de donner naissance à des clones dans la rate des souris irradiées à dose létale, cette protection étant due au maintien desdites cellules hors du cycle cellulaire, par l'inhibiteur.

Les documents publiés décrivent des techniques permettant d'obtenir des extraits plus ou moins purifiés contenant l'inhibiteur spécifique, mais dans tous les cas le produit finalement obtenu n'est pas homogène et les rendements sont peu satisfaisants. Il n'avait donc pas été possible jusqu'à présent d'isoler le principe actif et d'établir sa structure.

Or les demandeurs ont maintenant mis au point un nouveau procédé d'extraction qui permet d'obtenir une fraction active homogène à partir d'une matière biologique, notamment de moelle de veau foetal.

Ce procédé comprend essentiellement les étapes consistant à :
– broyer la matière de départ avec si nécessaire délipidation,
– mettre le produit obtenu en suspension dans du tampon à un pH voisin de 7, en présence d'un réactif réducteur soufré, notamment du dithioérythritol ou de préférence du mercaptoéthanol,
– centrifuger l'homogénat à environ 15 000 g pendant au moins 1 heure,
– soumettre le surnageant à une ultrafiltration sur membrane ayant une limite d'exclusion voisine de 10 000 daltons,
– soumettre l'ultrafiltrat obtenu à une chromatographie sur tamis moléculaire de type gel de polyacrylamide, avec élution par une solution diluée d'acide acétique,
– soumettre la fraction active recueillie à un fractionnement sur un support en phase inverse en silice greffée par des résidus aliphatiques, notamment en $C_{18}$, en répétant de préférence au moins une fois cette étape; et
– soumettre la nouvelle fraction active recueillie à une chromatographie liquide haute pression sur colonne en phase inverse en silice greffée par des résidus aliphatiques, notamment en $C_{18}$, pour obtenir une fraction active homogène.

La "fraction active" est identifiée par des tests biologiques, notamment tels que décrits dans la partie expérimentale qui suit.

Un exemple détaillé, non limitatif, de mise en oeuvre de ce procédé d'extraction est donné dans la partie expérimentale du présent mémoire.

2

La combinaison de différentes techniques d'analyse, notamment RMN, spectrométrie de masse et analyse du contenu en acides aminés par chromatographie liquide haute pression (HPLC) a permis de déterminer la structure du produit homogène ainsi isolé. Ce produit st un tétrapeptide acétylé de masse moléculaire 487, répondant à la formule :

Ser(N-Ac)-Asp-Lys-Pro-OH

ou

N-acétyl-séryl-aspartyl-lysyl-proline.

Les résultats décrits antérieurement ne permettaient pas de penser que l'activité observée des extraits plus ou moins purifiés obtenus pouvait être due à un tétrapeptide du type susindiqué.

En effet, on avait notamment constaté un effet protecteur du mercaptoéthanol sur la fraction inhibitrice qui semblait dû à la présence de groupes thiol dans la molécule d'inhibiteur, la présence de tels groupes ayant été établie dans la fraction inhibitrice relativement purifiée obtenue précédemment (voir IRCS Med. Sci. 12, (1984), 868-869),

De même, des résidus de sucres ayant été détectés, on pensait que le principe actif pouvait être de type glycopeptide, cette supposition étant confortée par le fait que l'inhibiteur en question peut être classé parmi les chalones ou anthormones et que la plupart des substances de ce type sont glycosylées.

L'invention a donc pour objet le tétrapeptide

Ser-Asp-Lys-Pro-OH

et ses dérivés de substitution par un ou plusieurs groupes protecteurs de peptides, identiques ou différents, couramment utilisés dans la chimie des peptides à usage biologique. Avantageusement, ces groupes sont choisis parmi acétyle, benzyle, méthyle et phényle. Le dérivé monoacétylé sur l'azote terminal est tout particulièrement préféré.

L'invention a également pour objet les sels pharmaceutiquement acceptables de ces composés.

Le tétrapeptide Ser-Asp-Lys-Pro-OH peut être obtenu par extraction à partir de matières biologiques, notamment à partir de foie ou de moelle de veau foetal.

Il peut notamment être obtenu par le procédé décrit de façon générale plus haut et plus en détail dans la partie expérimentale. Il est alors sous forme acétylée (en position N-terminale) et peut être utilisé tel quel ou bien être désacétylé selon des techniques classiques dans la chimie des peptides.

Le peptide selon l'invention et ses dérivés de substitution peuvent également être obtenus par synthèse peptidique, notamment en phase liquide.

La synthèse peptidique en phase liquide a avantageusement lieu par adjonctions successives à partir de l'extrémité C terminale des groupes aminoacides appropriés, convenablement substitués ou protégés sur leurs groupements réactifs, avec, lorsque cela est nécessaire, élimination des groupements protecteurs.

L'inhibiteur obtenu selon l'invention ne semble pas présenter de spécificité d'espèce (dans les essais décrits ici il est extrait de la moelle de foetus de veau et actif chez la souris) ; il est donc envisageable de l'utiliser, ainsi que ses dérivés de substitution, de même que leurs sels pharmaceutiquement acceptables, dans de nombreuses applications médicales et biologiques, chez l'homme et l'animal.

Ces composés peuvent être utilisés pour la protection de la moelle osseuse au cours des traitements anticancéreux par la chimiothérapie.

Dans ce cas ils sont avantageusement formulés à l'état de poudre et administrés, en présence d'adjuvants et/ou excipients usuels, par voie i.v ou s.c, au cours du traitement chimiothérapeutique. La fréquence des administrations et la quantité de produit administré dépendent essentiellement de la cinétique des cellules souches qui peut varier suivant l'agent thérapeutique, sa posologie et le protocole de son utilisation.

D'autres modes "d'administration" peuvent être envisagés, par exemple celui consistant à faire produire l'inhibiteur dans l'organisme du patient en ayant recours aux techniques du génie génétique, par exemple par l'intermédiaire de bactéries.

Le tétrapeptide et ses dérivés de substitution selon l'invention peuvent par ailleurs être utilisés pour l'obtention d'anticorps spécifiques pour le dosage du taux d'inhibiteur circulant chez les patients et la détermination de son rôle dans certaines maladies du système hématopoïétique, ainsi qu'au cours de greffes de moelle.

L'usage de ces anticorps, dans le cas d'hyper-production de l'inhibiteur liée à des pathologies, peut également être envisagé.

## I. Extraction du peptide N-acétyl-séryl-aspartyl-lysyl-proline.

La matière première est constituée de moelle osseuse de veau foetal conservée congelée. A chacune des étapes décrites ci-dessous, les fractions isolées sont additionnées de mercaptoéthanol à la concentration finale $10^{-2}$ M.

5 kg de tissu sont broyés au moyen d'un homogénéisateur de type Waring blender (3 fois, 60 sec.) dans 40 l de tampon phosphate $10^{-2}$M, pH 7,2 en présence de mercaptoéthanol $10^{-2}$ M à 4°C. La suspension est centrifugée à 15 000 g pendant 1h30.

Le surnageant recueilli est ultrafiltré sur membrane de type Sartorius 121 36 qui permet de séparer les molécules en fonction de leur poids moléculaire et dont la limite d'exclusion est de $10^4$ daltons.

L'ultrafiltrat est concentré 20 fois par évaporation sous vide au moyen d'un évaporateur de type "flash évaporateur" (LUWA). Le concentré est ensuite lyophilisé. Le rendement est de 16,5 g/kg et la fraction est active chez la souris à raison d'environ 20 mg/souris (activité déterminée selon l'un ou l'autre des protocoles décrits plus loin). La poudre lyophilisée (50 g) est ensuite redissoute dans 200 ml d'une solution d'acide acétique $10^{-2}$M (pH 3) puis centrifugée (10 min, 15 000 g). Le surnageant est chromatographié sur une colonne de tamis moléculaire de type Biogel P-2, ayant environ 0,07 à 0,04 mm "d'ouverture de maille°" (200-400 mesh) (BIO-RAD), de dimensions 12,5 X 100 cm, et élué avec 20 l d'acide acétique $10^{-2}$M (débit 400 ml/heure).

La fraction active est éluée au rapport d'élution Ve/Vo 1,2-1,8, Ve étant le volume d'élution et Vo le volume mort de la colonne. Le rendement est de 250 mg/kg de matière de départ et la fraction est active chez la souris à raison d'environ 5 µg/souris.

La fraction active (10 mg) est ensuite dissoute dans 1 ml d'eau, puis fractionnée successivement sur deux cartouches de support en phase inverse en silice greffée avec des résidus aliphatiques en $C_{18}$ de type Sep-pak C-18 (WATERS). Les cartouches sont éluées dans l'ordre avec :
- 2 ml de $H_2O$
- 2 ml d'un mélange $H_2O/CH_3OH$(50/50)
- 2 ml de $CH_3OH$

La fraction active éluée avec le mélange $H_2O/CH_3OH$ (50/50) est ensuite concentrée sous vide, à une température d'environ 20°C sur un appareil de type Speed Vac, puis lyophilisée.

La purification est poursuivie par chromatographie liquide haute pression sur une colonne analytique en phase inverse en octadécyl-silice de type ODS-Hypersil C-18, (250 X 4,6 mm) 5µ (SFCC). L'élution est effectuée avec le mélange $H_2O,CF_3COOH$ 0,1 %-MeOH (80-20) ou le mélange $H_2O,CF_3COOH$ 0,1 %-$CH_3CN$ (95-5) avec un débit de 1 ml/min. La détection des fractions est effectuée par mesure de l'absorption à 215 nm.

Les résultats obtenus sont les suivants :

1. Solvant ($H_2O,CF_3COOH$ 0,1 %)/$CH_3OH$, [80/20] débit 1 ml/min ; temps de rétention : 6 min : 1 pic homogène.
2. Solvant $H_2O,CF_3COOH$ 0,1 % $CH_3CN$, [95/5] débit 1 ml/min ; temps de rétention : 18 min : 1 pic homogène.

On constate que ce procédé permet d'obtenir un produit homogène dont la structure est déterminée comme indiqué plus loin.

Le rendement global est de 60 µg/kg de matière de départ.

La fraction est active chez la souris à raison d'environ 100 ng/souris.

## II. Détermination de la structure du principe actif :

### 1. Analyse des acides aminés :

Après hydrolyse acide, l'analyse par HPLC des dérivés formés par réaction avec l'orthophtaldialdéhyde permet de constater la présence de lysine, acide aspartique et sérine. L'analyse du produit à l'aide d'un analyseur d'acides aminés, après hydrolyse acide et oxydation par l'hypochlorite de sodium permet de constater la présence de proline et de confirmer la présence d'acide aspartique, sérine et lysine.

### 2. Spectroscopie RMN :

La RMN à une et à deux dimensions ($H_2O$ et $D_2O$) montre la présence de quatre acides aminés (proline, lysine, acide aspartique, sérine) et d'un groupement acétyle. Le $NH_2$ de la chaîne latérale de la lysine et le OH de la sérine sont libres, les $NH_2$ des parties acides aminés de la sérine, de la lysine et de l'acide aspartique sont substitués une fois. La structure probable est celle d'un peptide acétylé sur le $NH_2$ terminal.

### 3. Spectyométrie de masse :

Les techniques de spectrométrie de masse (SM) en FAB (Fast Atom Bombardment) ont montré que ce peptide a une masse moléculaire (M + 1) de 488.

Une étude de la séquence des acides aminés par une variante de la spectrométrie de masse, à savoir la technique dite MS-MS, a permis d'établir que la structure primaire complète du peptide est :

$$Ser(N-Ac)-Asp-Lys-Pro-OH$$

### III. Description des essais biologiques :

L'activité biologique des fractions est mesurée par des essais in vivo d'inhibition de l'entrée en cycle des CFU-S induite par une injection d'Ara-C.

Les CFU-S de la souris, normalement quiescentes, entrent en cycle après une injection d'Ara-C. Les demandeurs ont montré que lorsque l'inhibiteur est injecté 6 heures après la drogue, il empêche l'entrée des CFU-S en synthèse de ADN (phase S).

Le nombre des CFU-S est déterminé par la technique de J.E.Till et E.A. McCulloch, Radiat. Res., 14, (1961), 213-222, dont le principe repose sur la capacité des CFU-S de former des colonies macroscopiques dans la rate. Ces colonies sont des clones issus d'une CFU-S.

La proportion des CFU-S en phase S est déterminée par la méthode de A.J. Becker et coll., Blood, 26, (1965), 296-308, basée sur le principe des suicides cellulaires. L'incubation des cellules avec une solution de thymidine tritiée (précurseur de l'ADN) conduit à la mort sélective des cellules en phase de synthèse d'ADN par intégration d'une dose létale de radioactivité (tritium) dans la molécule d'ADN.

Les essais sont effectués sur des souris SPF (Specific Pathogen Free) de souche Balb/C ou CBA, âgées de 2 à 3 mois.

1. Les groupes témoins reçoivent par voie i.p. le traitement suivant :

Temps 0 : Ara-C (10 mg - protocole I ou 20 mg - protocole II) dissous dans 0,2 ml de sérum physiologique ;
Temps 6 h : sérum physiologique 0,2 ml ;
Temps 8 h : sacrifice (protocole I) ;
Temps 12 h : sacrifice (protocole II).

2. Les groupes traités reçoivent par voie i.p :

Temps 0 : Ara-C (10 mg - protocole I ou 20 mg - protocole II) dissous dans 0,2 ml de sérum physiologique.
Temps 6 h : fraction à tester dissoute dans 0,2 ml de sérum physiologique.
Temps 8 h : sacrifice (protocole I).
Temps 12 h : sacrifice (protocole II).

```
                                    Protocole I        Protocole II

                                         ↓                  ↓
    O                          6         8                 12

                                                          heures

    I─────────────────────────I─────I─────────────────────I-

    ↑                         ↑
    Ara-C                     Inhibiteur

                                            Détermination

                                            du nombre de CFU-S

                                            en cycle.
```

Pour chacun des groupes, la moelle osseuse des animaux est collectée et mise en suspension dans 2 ml de milieu 199 (EUROBIO). Après numération et dilution appropriée, 2 aliquotes de $5 \times 10^6$ cellules nuclées sont incubées à 37°C pendant 20 min soit avec 1 ml de milieu 199 (Essai A), soit avec 1 ml de thymidine tritiée (200 µCi) (Essai B). Une suspension cellulaire de 0,2 ml contenant $8.10^4$ à $1,2.10^5$ cellules nuclées est injectée par voie intra-veineuse dans le sinus-rétro-orbital des animaux receveurs irradiés (9 Gy, 8 souris par point expérimental). Neuf jours plus tard les souris receveurs sont sacrifiées, leur rate prélevée et fixée dans le liquide de Bouin (acide picrique à 1 % : 720 g ; formol : 240 g ; acide acétique 4 g pour 1 l de liquide). Après quelques heures de fixation, les nodules visibles macroscopiquement sont dénombrés.

Si N et N′ sont les nombre moyens de nodules obtenus sur les rates des receveurs ayant reçu les cellules provenant des essais A et B respectivement, la proportion des CFU-S en phase de synthèse d'ADN est calculée par la formule :

$$\text{\% de CFU-S en S} = \frac{N-N'}{N} \times 100$$

Résultats :

1. Inhibition de l'entrée en cycle des CFU-S.

Au cours de chaque étape de purification, on isole une fraction active vis-à-vis de ce test. Toutefois une activité particulièrement intéressante est observée avec la fraction homogène obtenue selon l'invention, comme le montre le tableau suivant :

Inhibition de l'entrée en cycles des CFU-S par injection de 100 ng par souris de la fraction homogène obtenue selon l'invention, selon le protocole II.

## TABLEAU I

| Traitement | % de CFU-S en synthèse d'ADN |
|---|---|
| Ara-C (20 mg) | 43±9 |
| Ara-C (20 mg) + inhibiteur (100 ng) | 7±4,3 |

2. Activité sur la survie des animaux traités avec des doses létales d'Ara-C.

Les animaux reçoivent des doses fractionnées d'Ara-C ($4 \times 925$ mg/kg) aux temps 0, 7, 24 et 30 heures. La fraction testée est administrée en 1 fois 26 heures après la première injection d'Ara-C. La survie des animaux est estimée 15-30 jours après le traitement. Dans ce protocole comportant des injections répétées, létales d'Ara-C, il a été montré que l'administration concomitante d'inhibiteur permettait la survie définitive d'un grand nombre d'animaux. Les résultats obtenus sont comparables à ceux observés lorsque l'on procède à une greffe de moelle allogénique des animaux traités, au lieu d'utiliser l'inhibiteur.

## Protocole :

```
O            7                         24        26          30
                                                            heure

I————————I————————————————————I————————I——————————I

↑            ↑                          ↑          ↑          ↑

Ara-C        Ara-C                    Ara-C                 Ara-C

                                          Inhibiteur
```

### 3. Absence d'activité de la fraction inhibitrice sur la régression de tumeurs EMT6.

Le traitement des animaux porteurs de la tumeur EMT6 par 4 injections successives d'Ara-C aux temps 0,7, 24 et 30 heures suivies d'une injection de l'inhibiteur 26 heures après la première injection de l'Ara-C, n'empêche pas l'action curative de l'Ara-C vis-à-vis des cellules tumorales et permet la survie des animaux traités de façon comparable aux résultats obtenus suite à une greffe de moelle.

### 4. Toxicité

La fraction inhibitrice est dépourvue de toxicité vis-à-vis des CFU-S et n'entraîne aucune létalité chez les souris traitées, aux doses utilisées.

### IV. Synthèse en phase liquide de NAc-Ser-Asp-Lys-Pro-OH soit en écriture abrégée conventionnelle AcSDKP.

#### a) Synthèse de Boc-K(Z)-P-OH (I) :

A une solution de Boc-K(Z)-OSu(4,19 mmoles) dans du DMF est ajoutée une solution aqueuse de P (8,38 mmoles) dans laquelle ont été placés 1,15 ml de triéthylamine (8,38 mmoles). Le mélange est agité une nuit à température ambiante.

Après évaporation du solvant, le résidu est dissous dans l'acétate d'éthyle. La solution, lavée successivement par une solution d'acide citrique à 5 %, une solution de carbonate de sodium à 5 % et par l'eau, est séchée sur $MgSO_4$. Après évaporation du solvant, le produit ainsi obtenu est caractérisé :

– huile jaunâtre : 3,98 mmoles ; rendement : 95 %
– $[\alpha]_D = -38°(C=1,0 ; MeOH)$
Rf : 0,5 ($MeOH/CHCl_3$ : 1/9) ; 0,24 (EtOAc/MeOH : 8/1)
Les Rf sont déterminés par chromatographie sur couche mince.
– RMN réalisée en solution dans $CDCl_3$ : conforme.

note    : il est rappelé que
Boc    = tert-butyloxycarbonyle ;
Z       = benzyloxycarbonyle ;
Su      = sulfonamide ;
DMF    = diméthylformamide.

#### b) Synthèse de Boc-D(OBzl) -K(Z) -p-OH (II) :

A une solution de Boc-D(OBzl)-OSu (3,31 mmoles) dans le DMF est ajoutée une solution de TFA.K(Z)-P-OH (3,15 mmoles) dans le DMF contenant 915 µl de triéthylamine, soit 6,62 mmoles. La solution de TFA.K(Z)-P-OH est obtenue par traitement du produit (I) par une solution de TFA (10 équivalents) et d'anisol (1 équivalent) pendant 1 heure à 0°C. Elle est lavée à l'hexane puis à l'éther avant d'être utilisée pour l'étape suivante. Le mélange ainsi constitué est mis sous agitation une nuit à température ambiante. Après évaporation du solvant, le résidu est solubilisé dans l'acétate d'éthyle et lavé comme en a) avant d'être caractérisé.

– huile : 2,17 mmoles ; rendement : 70 %

– $[\alpha]_D$ = 20°(C=1,2 ; MeOH)

Rf : 0,23 (MeOH/CHCl$_3$ : 1/9) ;

– RMN réalisée en solution dans CDCl$_3$ : conforme.

Note : il est rappelé que

TFA = acide trifluoroacétique et

Bzl = benzyle.

c) Synthèse de Boc-S(Bzl)-D(OBzl)-K(Z)-P-OH (III) :

Selon la même séquence qu'en b), 1,47 mmoles de Boc-S(Bzl)-Osu sont ajoutées à 1,47 mmoles de TFA.D(OBzl)-K(Z)-P-OH.

Le produit obtenu présente les caractéristiques suivantes :

– huile : 1,31 mmoles ; rendement : 89 %

– $[\alpha]_D$ = -26,7°(C=1,0 ; MeOH)

Rf : 0,29 (MeOH/CHCl$_3$ : 1/9) ;

– RMN réalisée en solution dans CDCl$_3$ additionné d'une petite quantité de MeOD pour améliorer la solubilité et la résolution des spectres : conforme.

d) Synthèse de Acs(Bzl) -D(OBzl) -K(Z) -P-OH (IV) :

Après élimination du Boc N-terminal du produit (III), le nouveau groupement bloquant est ajouté en une seule étape. Le produit final, après lavages, présente les caractéristiques suivantes lorsqu'on met en oeuvre 1 mmole de TFA.S(Bzl)D(OBzl)K(Z)POH :

– huile : 0,77 mmole ; rendement : 77 %

– $[\alpha]_D$ = -22,0°(C=1,0 ; MeOH)

Rf : 0,12 (CHCl$_3$/MeOH : 9/1) ; 0,54 (EtOAc/MeOH/CH$_3$COOH ; 16/3/1)

– RMN réalisée en solution dans CDCl$_3$ additionné de MeOD : conforme.

e) Obtention de Ac-S-D-K-P-OH (V) :

Le produit (IV) en solution dans le méthanol est soumis, après addition de charbon palladié, à une hydrogénolyse. Une fois la réaction terminée, le méthanol est évaporé et le résidu caractérisé.

Ce produit final qui se présente après lyophilisation, sous forme d'un solide blanc, est caractérisé par les mêmes techniques physico-chimiques que le peptide naturel, à savoir :

– analyse d'acides aminés ;

– chromatographie liquide haute performance;

– RMN en solution dans D$_2$O ; et

– spectrométrie de masse FAB et FAB (MS-MS).

Dans toutes ces techniques, on constate une bonne correspondance des résultats obtenus pour le produit synthétique V avec ceux obtenus pour le produit naturel.

En particulier, les spectres RMN du proton des peptides naturel et synthétique sont totalement superposables, de même que les spectres de masse.

L'analyse de ce tétrapeptide synthétique par HPLC en phase inverse sur colonne analytique ODS-Hypersil C 18, dans des conditions isocratiques, avec le mélange CH$_3$CN/H$_2$O, TFA à 0,1 % [4,5/95,5], avec une détection à 215 nm, permet de constater que le pic à 18 minutes, à 25°C comporte un léger épaulement qui apparaît plus nettement dans la première partie du pic à 24 minutes, à 15°C. Ce pic à 24 minutes, à 15°C peut être dédoublé en un pic de faible intensité à 22 minutes, correspondant à l'épaulement précédent, et en un pic à 24 minutes. Le produit correspondant à ce pic possède les propriétés biologiques du peptide naturel.

Il a été soumis aux tests décrits précédemment en relation avec l'inhibiteur naturel extrait selon l'invention.

Les pourcentages de CFU-S en phase de synthèse d'ADN obtenus sont rassemblés dans le tableau II qui suit.

## TABLEAU II

| Dose de tétrapeptide synthétique/souris | | Témoin | ARA-C | ARA-C + tétrapeptide synthétique |
|---|---|---|---|---|
| 1. | 100 ng | 20 | 44 | 12 |
| 2. | 100 ng | 10 | 30 | 1 |
| 3. | 100 ng | – | 55 | 17 |
| 4. | 100 ng | O | 26 | 6 |
| 5. | 50 ng | – | 35 | 13 |
| 6. | 25 ng | O | 48 | 19 |
| 7. | 100 ng | O | 35 | 10 |
| 8. | 100 ng | O | 36 | 6 |
| moyenne | | 5 | 38,7 | 10,5 |

Les essais 1, 2 et 3 ont été réalisés avec le tétrapeptide obtenu par une première synthèse, les essais 4, 5, 6, 7 et 8 ont été réalisés avec le tétrapeptide obtenu par une deuxième synthèse.

Les résultats obtenus sont à rapprocher de ceux obtenus avec le produit naturel et qui figurent dans le tableau I d'une part, et dans le tableau III, d'autre part.

Ces derniers résultats ont été obtenus avec le produit naturel extrait de deux lots différents A et B de moelle osseuse.

## TABLEAU III

| Lot de moelle osseuse | dose/ souris | témoin | ARA-C | ARA-C + peptide naturel |
|---|---|---|---|---|
| A (5 exp.) | 100 ng | 6,5±13,9 | 40,7±9,3 | 12,6±14,8 |
| B (2 exp.) | 100 ng | 11,9±16,3 | 47,4±9 | 2,99±13 |
| moyenne | | 9,2 | 44,1 | 7,8 |

L'examen comparatif des tableaux I et III, d'une part et II, d'autre part permet de constater que le peptide synthétique présente une activité tout à fait similaire à celle obtenue avec le peptide naturel.

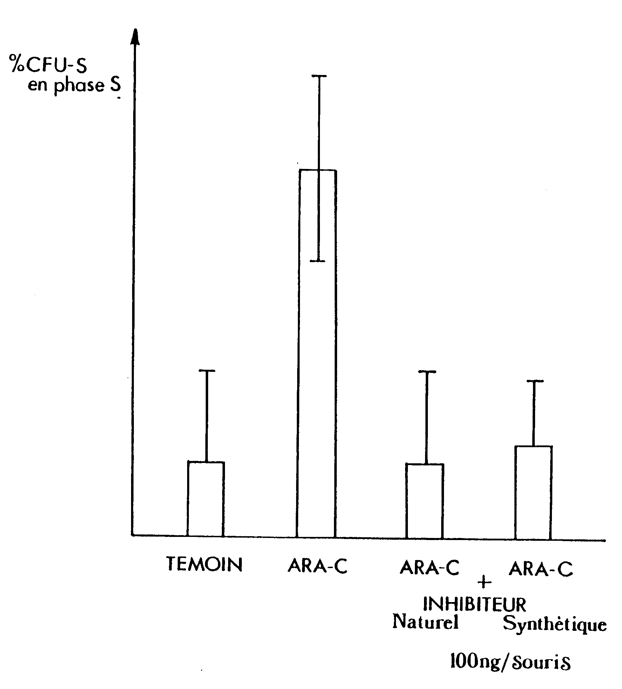

Les résultats obtenus sont représentés schématiquement sur la figure unique annexée.

**Revendications**

1.  Tétrapeptide répondant à la formule :

Ser-Asp-Lys-Pro-OH

et ses dérivés de substitution par un ou plusieurs groupes protecteurs de peptides, identiques ou différents couramment utilisés dans la chimie des peptides à usage biologique, ainsi que leurs sels pharmaceutiquement acceptables.

2.  Tétrapeptide selon la revendication 1, caractérisé en ce que le ou les groupes substituants sont choisis parmi acétyle, benzyle, méthyle et phényle.

3.  Tétrapeptide répondant à la formule :

Ser-(N-Ac)-Asp-Lys-Pro-OH

ainsi que ses sels pharmaceutiquement acceptables.

4.  Procédé pour l'extraction du tétrapeptide selon la revendication 3, à partir de matières biologiques, notamment à partir de foie ou de moelle de veau foetal, caractérisé en ce qu'il comprend essentiellement les étapes consistant à :
    – broyer la matière de départ avec si nécessaire délipidation,
    – mettre le produit obtenu en suspension dans du tampon à un pH voisin de 7, en présence d'un réactif réducteur soufré, notamment du dithioérythritol ou de préférence du mercaptoéthanol,
    – centrifuger l'homogénat à environ 15 000 g pendant au moins 1 heure,
    – soumettre le surnageant à une ultrafiltration sur membrane ayant une limite d'exclusion voisine de 10 000 daltons,
    – soumettre l'ultrafiltrat obtenu à une chromatographie sur tamis moléculaire de type gel de polyacrylamide, avec élution par une solution diluée d'acide acétique,
    – soumettre la fraction active recueillie à un fractionnement sur un support en phase inverse en silice greffée par des résidus aliphatiques, notamment en $C_{18}$, en répétant de préférence au moins une fois cette étape; et
    – soumettre la nouvelle fraction active recueillie à une chromatographie liquide haute pression sur colonne en phase inverse en silice greffée par des résidus aliphatiques, notamment en $C_{18}$, pour obtenir une fraction active homogène.

5.  Procédé pour la synthèse du tétrapeptide selon l'une des revendications 1 à 3, caractérisé en ce qu'il consiste essentiellement à ajouter successivement en phase liquide, à partir de l'extrémité C terminale, les groupes amino-acides appropriés, convenablement substitués ou protégés sur leurs groupements réactifs et, lorsque cela est nécessaire, à éliminer les groupements protecteurs.

6.  Médicament, caractérisé en ce qu'il comprend, en tant que principe actif, au moins un composé selon l'une des revendications 1 à 3.

7.  Médicament, notamment pour la protection de la moelle osseuse au cours de traitements anticancéreux par la chimiothérapie, caractérisé par le fait qu'il se présente sous la forme d'une poudre destinée à l'administration par voie i.v ou s.c en présence d'adjuvants et/ou excipients usuels, contenant, en tant que principe actif, au moins un composé selon l'une des revendications 1 à 3.

8.  Utilisation des composés selon l'une des revendications 1 à 3 pour la production d'anticorps spécifiques.


**Claims**

1.  Tetrapeptide of the general formula

Ser-Asp-Lys-Pro-OH

and its substitution derivatives by one or several groups protecting the peptides, identical or different, currently used in the chemistry of peptides for biological use, as well as their pharmaceutically acceptable salts.

2.  Tetrapeptide according to Claim 1, characterized in that the one or more substituent groups are selected from the group consisting of acetyl, benzyl, methyl and phenyl.

3. Tetrapeptide of the formula:

$$Ser-(N-Ac)-Asp-Lys-Pro-OH$$

as well as its pharmaceutically acceptable salts.

4. Process for the extraction of the tetrapeptide according to Claim 3, from biological materials, particularly from fetal calf liver or marrow, characterized in that it comprises essentially the steps consisting of:
– grinding the starting material with delipidation if necessary,
– suspending the product obtained in a buffer at a pH close to 7, in the presence of a sulphur-containing reducing reagent, particularly dithioerythritol or, preferably, mercaptoethanol,
– centrifuging the homogenizate at about 15,000g for at least one hour,
– subjecting the supernatant to ultrafiltration on a membrane having an exclusion limit close to 10,000 daltons,
– subjecting the ultrafiltrate obtained to chromatography on a molecular sieve of the polyacrylamide gel type, with elution by a dilute solution of acetic acid,
– subjecting the active fraction collected to fractionation on a reverse phase support of silica bonded with aliphatic residues, particularly with 18C, repeating this step preferably at least once; and
– subjecting the novel active fraction collected to high pressure liquid chromatography on a reverse phase column of silica bonded with aliphatic residues, particularly with 18C, to obtain a homogeneous active fraction.

5. Process for the synthesis of the tetrapeptide according to one of Claims 1 to 3, characterized in that it essentially consists of adding successively in liquid phase, from the C terminal end, the suitable amino acid groups, suitably substituted or protected on their reactive groups, and when this is necessary, eliminating the protective groups.

6. Medicine, characterized in that it comprises, as active principle, at least one compound according to one of Claims 1 to 3.

7. Medicine, particularly for the protection of the bone marrow in the course of anti-cancer treatment by chemotherapy, characterized in that it is in the form of a powder in tended for i.v. or s.c. administration in the presence of usual adjuvants and/or excipients, containing as active principle, at least one compound according to one of Claims 1 to 3.

8. Use of the compounds according to one of Claims 1 to 3 for the production of specific antibodies.


**Patentansprüche**

1. Tetrapeptid, entsprechend der Formel:

$$Ser-Asp-Lys-Pro-OH$$

und seine Substitutionsderivate mit einer oder mehreren, identischen oder verschiedenen Peptidschutzgruppen, die üblicherweise in der Peptidchemie für den biologischen Gebrauch benutzt werden, sowie ihre pharmazeutisch annehmbaren Salze.

2. Tetrapeptid nach Anspruch 1, dadurch gekennzeichnet, daß die Substituentengruppe oder die Substituentengruppen ausgewählt sind aus Acetyl, Benzyl, Methyl und Phenyl.

3. Tetrapeptid, entsprechend der Formel:

$$Ser-(N-Ac)-Asp-Lys-Pro-OH$$

sowie seine pharmazeutisch annehmbaren Salze.

4. Verfahren zur Extraktion des Tetrapeptids nach Anspruch 3 aus biologischen Materialien, insbesondere aus fötaler Kalbsleber oder fötalem Kalbsmark, dadurch gekennzeichnet, daß es im wesentlichen die Schritte bestehend aus:

– das Ausgangsmaterial, falls notwendig mit Delipidierung, zu zerkleinern,

– das erhaltene Produkt in einem Puffer bei einem pH-Wert nahe 7 in Gegenwart eines reaktiven schwefelhaltigen Reduktionsmittels, insbesondere von Dithioerythrit oder vorzugsweise von Mercaptoethanol, zu suspendieren,

– das Homogenisat bei ungefähr 15 000 g während mindestens 1 Stunde zu zentrifugieren,

– den Überstand einer Ultrafiltration durch eine Membran mit einer Ausschlußgrenze nahe 10 000 Dalton zu unterwerfen,

– das erhaltene Ultrafiltrat einer Chromatographie an einem Molekularsieb des Polyacrylamidgeltyps mit Elution durch eine verdünnte Essigsäurelösung zu unterwerfen,

– die gewonnene aktive Fraktion einer Fraktionierung auf einem Umkehrphasenträger aus mit aliphatischen Resten, insbesondere mit $C_{18}$ gepfropfter Kieselerde zu unterwerfen, wobei dieser Schritt vorzugsweise mindestens einmal wiederholt wird, und

– die gewonnene neue aktive Fraktion einer Hochdruckflüssigkeitschromatographie an einer Umkehrphasensäule aus mit aliphatischen Resten, insbesondere mit $C_{18}$ gepfropfter Kieselerde zu unterwerfen, um eine homogene aktive Fraktion zu erhalten, umfaßt.

5. Verfahren für die Synthese des Tetrapeptids nach einem der Ansprüche 1 bis 3, dadurch gekennzeichnet, daß es im wesentlichen daraus besteht, nach und nach in flüssiger Phase vom C-terminalen Ende ausgehend die geeigneten Aminosäuregruppen, die an ihren reaktiven Gruppen passend substituiert oder geschützt sind, zuzugeben und, wenn dies notwendig ist, die Schutzgruppen zu eliminieren.

6. Medikament, dadurch gekennzeichnet, daß es als aktives Element mindestens einen Beständteil gemäß einem der Ansprüche 1 bis 3 umfaßt.

7. Medikament, insbesondere für den Schutz des Knochenmarks im Verlaufe von Anti-Krebsbehandlungen durch die Chemotherapie, gekennzeichnet durch die Tatsache, daß es in Form eines Puders vorkommt, der für die Verabreichung über den i.v.- oder s.c.-Weg in Gegenwart üblicher Hilfsstoffe und/oder Bindemittel bestimmt ist, enthaltend als aktives Element mindestens einen Bestandteil gemäß einemder Ansprüche 1 bis 3.

8. Verwendung der Zusammensetzungen gemäß einem der Ansprüche 1 bis 3 für die Herstellung spezifischer Antikörper.

EP 0 316 322 B1